# EUROPEAN PATENT APPLICATION

(11) **EP 3 254 674 A1**
(43) Date of publication of application: **13.12.2017**
(21) Application number: 16173539.4
(22) Date of filing: 08.06.2016
(51) Int. Cl.: A61K 9/14, A61K 31/44, A61P 7/02

(54) **PHARMACEUTICAL COMPOSITIONS OF BETRIXABAN MALEATE**

(71) Applicant: SANDOZ AG, 4056 Basel (CH)
(72) Inventor: MARTIN, Nolwenn, 6250 Kundl (AT)
(74) Representative: Prüfer & Partner mbB Patentanwälte · Rechtsanwälte

(57) **Abstract**

The present invention relates to a solid dispersion comprising amorphous betrixaban maleate and a pharmaceutically acceptable carrier selected from the group consisting of organic polymers and silicon-based inorganic compounds, and to a process for preparing the same. It also relates to a pharmaceutical composition comprising said solid dispersion and one or more pharmaceutically acceptable excipients. The invention also concerns the use of said pharmaceutical compositions as a medicament, in particular for the prevention and the treatment of disease conditions characterized by undesired thrombosis.

## Description

### FIELD OF THE INVENTION

The present invention relates to a solid dispersion comprising amorphous betrixaban maleate and a process for preparing the same. It also relates to a pharmaceutical composition comprising said solid dispersion and one or more pharmaceutically acceptable excipients. The invention also concerns the use of said pharmaceutical compositions as a medicament, in particular for the prevention and the treatment of disease conditions characterized by undesired thrombosis.

### BACKGROUND OF THE INVENTION

Betrixaban, N-(5-chloropyridin-2-yl)-2-([4-(N,N-dimethylcarbamimidoyl)benzoyl]amino)-5-methoxybenzamide, is a Factor Xa inhibitor under clinical development for the prevention and treatment of venous thrombosis. It can be represented by the following chemical structure:

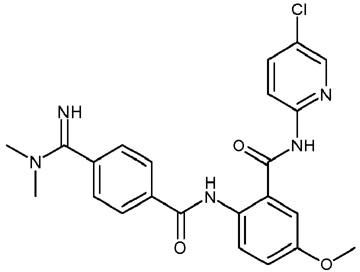

WO 2007/056517 A2 discloses betrixaban salts and a process for their preparation. In particular betrixaban maleate (1:1) and a crystalline polymorphic form thereof called Form I is disclosed. WO2012/031017 discloses two further crystalline forms of betrixaban maleate: Form II - the thermodynamically most stable form - and Form III, which is a channel hemihydrate of Form II. Form II was first produced inadvertently while attempting to make Form I and reversibly converts to Form III at relative humidity above 25%. The process for the preparation of Form I is therefore not reliable and crystalline Forms II and III are moisture sensitive since they reversible convert to each other depending on the relative humidity.

While amorphous solids have increased solubility and an accelerated dissolution rate in comparison to their crystalline counterparts, crystalline solid forms of an active pharmaceutical ingredient are often preferred for the preparation of pharmaceutical compositions for various reasons such as stability with respect to the solid form or chemical stability, or ease of handling during the formulation process. Amorphous solid are difficult to stabilize in a pharmaceutical composition and have a tendency to convert, in part or completely, to crystalline material over time. Such conversion is highly undesired, because it results in a change over time of physical properties, such as solubility or dissolution speed. As a consequence, slower dissolution and lower bioavailability of the drug are consciously accepted and pharmaceutical compositions, which are maybe not optimal with regard to these parameters, are produced and marketed.

Therefore the object of the present invention is the provision of a pharmaceutical composition of betrixaban maleate that allows fast dissolution and high bioavailability, while at the same time retaining its physical and chemical properties over time, for example throughout its shelf life. A further object of the present invention is the provision of a pharmaceutical composition of betrixaban maleate, which is prepared using a reliable process and whose physical form is stable over the whole range of relative humidity.

### SUMMARY OF THE INVENTION

The present inventors have managed to prepare stable pharmaceutical compositions comprising amorphous betrixaban maleate despite its high tendency to crystallize. The pharmaceutical compositions of the present invention are advantageous compared to pharmaceutical compositions comprising crystalline betrixaban maleate in that their faster dissolution can lead to a faster onset of action, which can be beneficial for the prevention and the treatment of disease conditions characterized by undesired thrombosis.

It was surprisingly found that amorphous betrixaban maleate could be stabilized in the context of a solid dispersion comprising a pharmaceutically acceptable carrier selected from the group consisting of organic polymers and silicon-based inorganic compounds. Therefore, the present invention relates to a solid dispersion comprising amorphous betrixaban maleate and a pharmaceutically acceptable carrier selected from the group consisting of organic polymers and silicon-based inorganic compounds and to a process for its preparation. It also relates to a pharmaceutical composition comprising said solid dispersion and one or more pharmaceutically acceptable excipients. In addition, the present invention relates to the use of said pharmaceutical composition as a medicament, in particular for the treatment of disease conditions characterized by undesired thrombosis.

### Abbreviations

- PXRD: powder X-ray diffractogram
- DSC: differential scanning calorimetry
- TGA: thermogravimetric analysis
- DVS: dynamic vapour sorption
- Δm: mass difference
- RH: relative humidity

### Definitions

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see "Fundamentals of Powder Diffraction and Structural Characterization of Materials " by Vitalij K. Pecharsky and Peter Y. Zavalij, Kluwer Academic Publishers, 2003, page 3).

As used herein, the term "substantially amorphous" means that the amorphous form includes at most 20%, preferably at most 10%, more preferably at most 5%, even more preferably at most 2% and most preferably at most 1% by weight of any crystalline form.

The term "solvate" as used herein refers to a solid, where one or more organic solvent(s) is/are coordinated in or accommodated by the crystal structure.

The term "hydrate" as used herein refers to a solid, where water is coordinated in or accommodated by the crystal structure.

As used herein, the term "about" means within a statistically meaningful range of a value. Such a range can be within an order of magnitude, typically within 10%, more typically within 5%, even more typically within 1% and most typically within 0.1 % of the indicated value or range. Sometimes, such a range can lie within the experimental error, typical of standard methods used for the measurement and/or determination of a given value or range.

As used herein, the term "silicate" refers to naturally occurring or synthesized compounds containing an anionic silicon compound, preferably an oxide.

As used herein, the term "silica" refers to naturally occurring or synthesized silica.

As used herein, the term "silicon-based inorganic adsorbent" refers to a silicon-based inorganic compound having a high porosity and a large surface area that enables it to adsorb high loads of oil or water.

As used herein, the term "betrixaban maleate Form I" or simply "Form I" refers to crystalline betrixaban maleate characterized by having a powder X-ray diffraction pattern comprising peaks at 2-theta angles of 4.9 ± 0.2°, 9.7 ± 0.2°, 13.8 ± 0.2°, 14.1 ± 0.2°, 15.2 ± 0.2°, 17.6 ± 0.2°, 18.5 ± 0.2°, 20.8 ± 0.2°, 21.6 ± 0.2°, 22.7 ± 0.2°, 24.1 ± 0.2°, 26.3 ± 0.2° and 26.8 ± 0.2°, when measured at a temperature in the range of from 20 to 30°C with Cu-Kalpha_{1,2} radiation having a wavelength of 0.15419 nm (according to WO 2007/056517 A2); alternatively the term "betrixaban maleate Form I" or simply "Form I" refers to crystalline betrixaban maleate characterized by having a powder X-ray diffraction pattern comprising peaks at 2-theta angles 5.0 ± 0.2°, 10.0 ± 0.2°, 13.9 ± 0.2°, 14.0 ± 0.2°, 15.0 ± 0.2°, 17.5 ± 0.2°, 18.0 ± 0.2°, 20.0 ± 0.2°, 26.5 ± 0.2° (Form A, according to WO 2011/084519 A1), when measured at a temperature in the range of from 20 to 30°C with Cu-Kalpha_{1,2} radiation having a wavelength of 0.15419 nm.

As used herein, the term "betrixaban maleate Form II" or simply "Form II" refers to crystalline betrixaban maleate characterized by having a powder X-ray diffraction pattern comprising peaks at 2-theta angles of 5.0 ± 0.1°, 9.7 ± 0.1°, 10.1 ± 0.1°, 15.3 ± 0.1°, 17.5 ± 0.1° and 19.6 ± 0.1°, when measured at a temperature in the range of from 20 to 30°C with Cu-Kalpha_{1,2} radiation having a wavelength of 0.15419 nm.

As used herein, the term "betrixaban maleate Form III" or "Form III" refers to crystalline betrixaban maleate characterized by having a powder X-ray diffraction pattern comprising peaks at 2-theta angles of 2.2 ± 0.1°, 4.9 ± 0.1°, 10.0 ± 0.1 °, 15.1 ± 0.1°, 17.4 ± 0.1°, and 22.4 ± 0.1°, when measured at a temperature in the range of from 20 to 30°C with Cu-Kalpha_{1,2} radiation having a wavelength of 0.15419 nm.

### BRIEF DESCRIPTION OF THE DRAWINGS

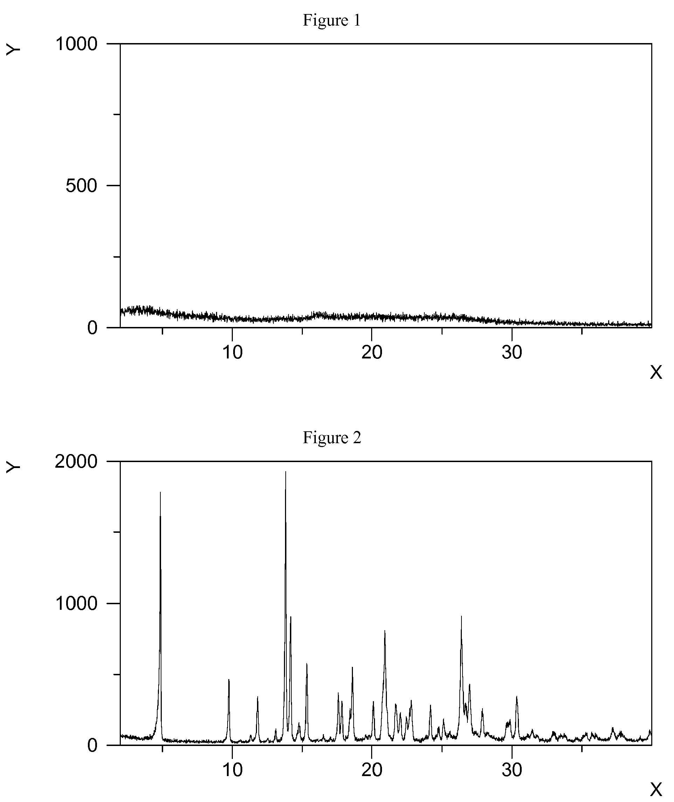

**Figure 1** illustrates a representative PXRD of amorphous betrixaban maleate prepared according to the procedure described in Comparative Example 1. The X-axis shows the 2-theta angle /° and the Y-axis shows the intensity / counts.
**Figure 2** illustrates a representative PXRD of amorphous betrixaban maleate prepared according to the procedure described in Comparative Example 1 after one week at 40°C and 75% RH. The X-axis shows the 2-theta angle /° and the Y-axis shows the intensity / counts.

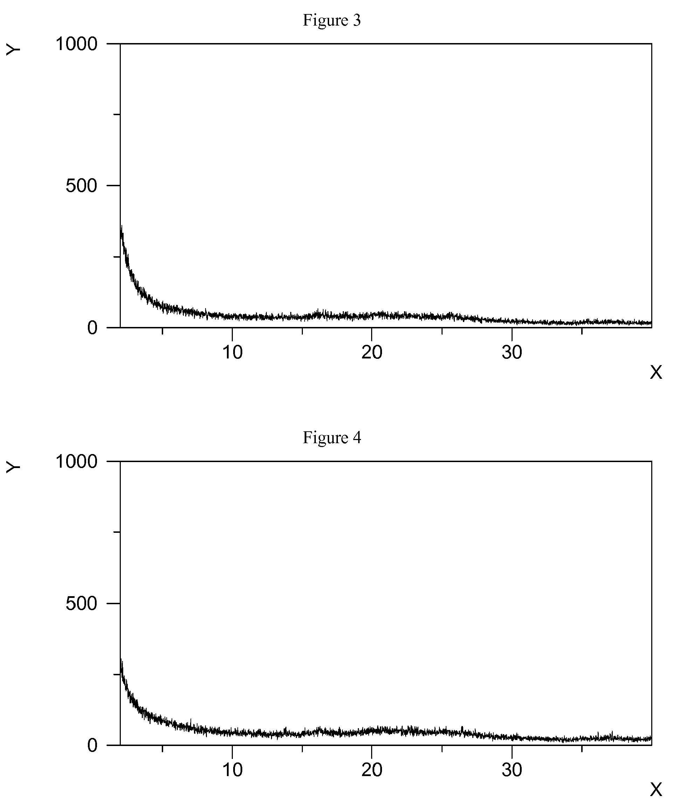

**Figure 3** illustrates a representative PXRD of amorphous solid dispersion of betrixaban maleate and Neusilin^{®} UFL2 prepared according to the procedure described in Example 1-1. The X-axis shows the 2-theta angle /° and the Y-axis shows the intensity / counts.
**Figure 4** illustrates a representative PXRD of amorphous solid dispersion of betrixaban maleate and Neusilin^{®} UFL2 prepared according to the procedure described in Example 1-1 after storage at 40°C and 75% relative humidity for six weeks. The X-axis shows the 2-theta angle /° and the Y-axis shows the intensity / counts.

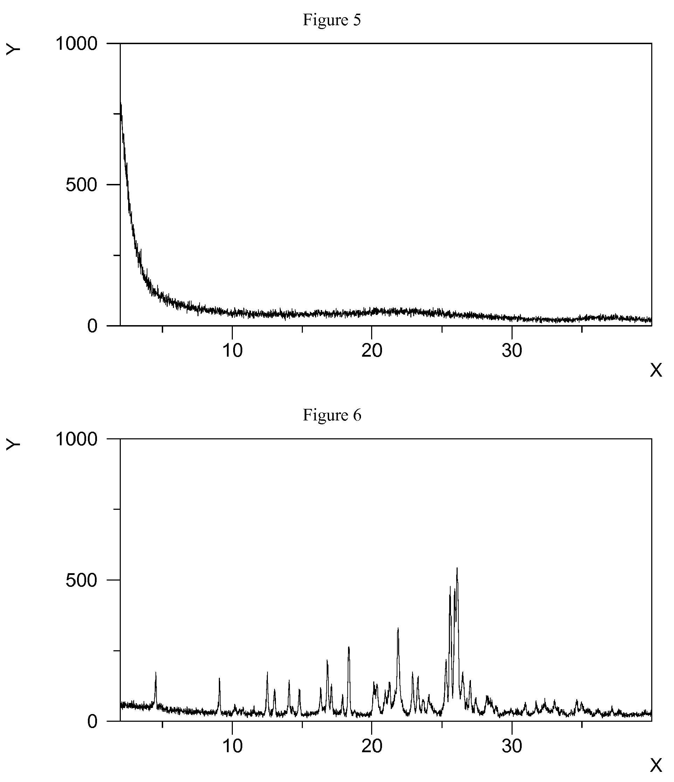

**Figure 5** illustrates a representative PXRD of amorphous solid dispersion of betrixaban maleate and Neusilin^{®} UFL2 prepared according to the procedure described in Example 1-3 after storage at 40°C and 75% relative humidity for six weeks. The X-axis shows the 2-theta angle /° and the Y-axis shows the intensity / counts.
**Figure 6** illustrates a representative PXRD of betrixaban maleate dichloromethane solvate prepared according to Example 2. The X-axis shows the 2-theta angle /° and the Y-axis shows the intensity / counts.

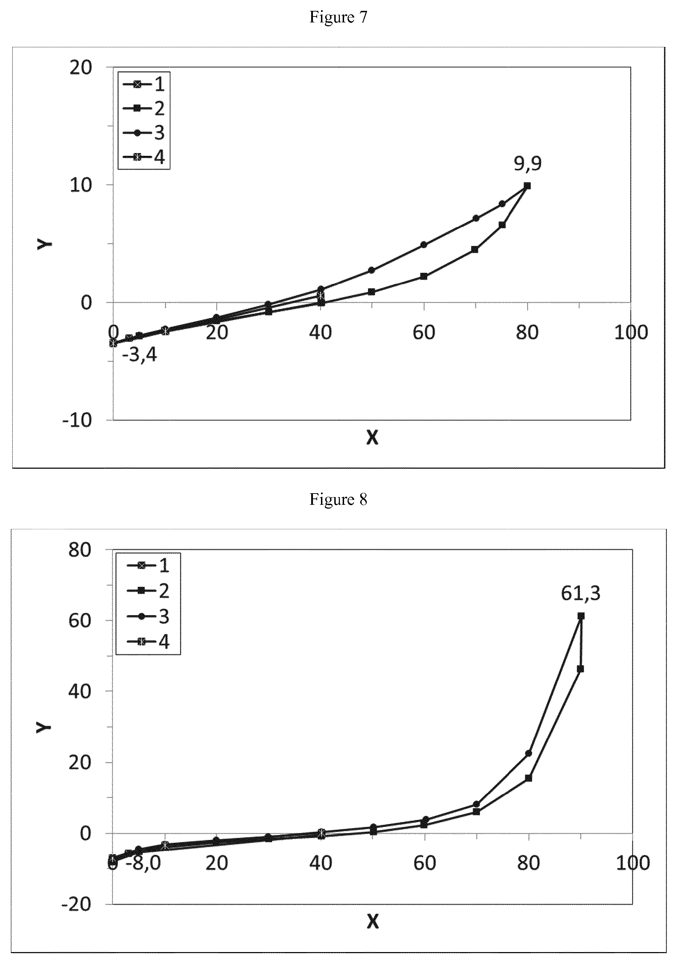

**Figure 7** illustrates a representative DVS isotherm of hydroxypropyl methylcellulose (Methocel E5, used for example in Example 3-2A), recorded as described in Example part. The X-axis shows the relative humidity /% and the Y-axis shows the mass difference Δm / %. The Δm(desorption) values are obtained from the desorption isotherm (symbols: ●), the Δm(adsorption) values are obtained from the adsorption isotherm (symbols: ■).
**Figure 8** illustrates a representative DVS isotherm of magnesium aluminometasilicate Neusilin^{®} UFL2 (used for example in Example 4-1A), recorded as described in Example part. The X-axis shows the relative humidity / % and the Y-axis shows the mass difference Δm / %. The Δm(desorption) values are obtained from the desorption isotherm (symbols: ●), the Δm(adsorption) values are obtained from the adsorption isotherm (symbols: ■).

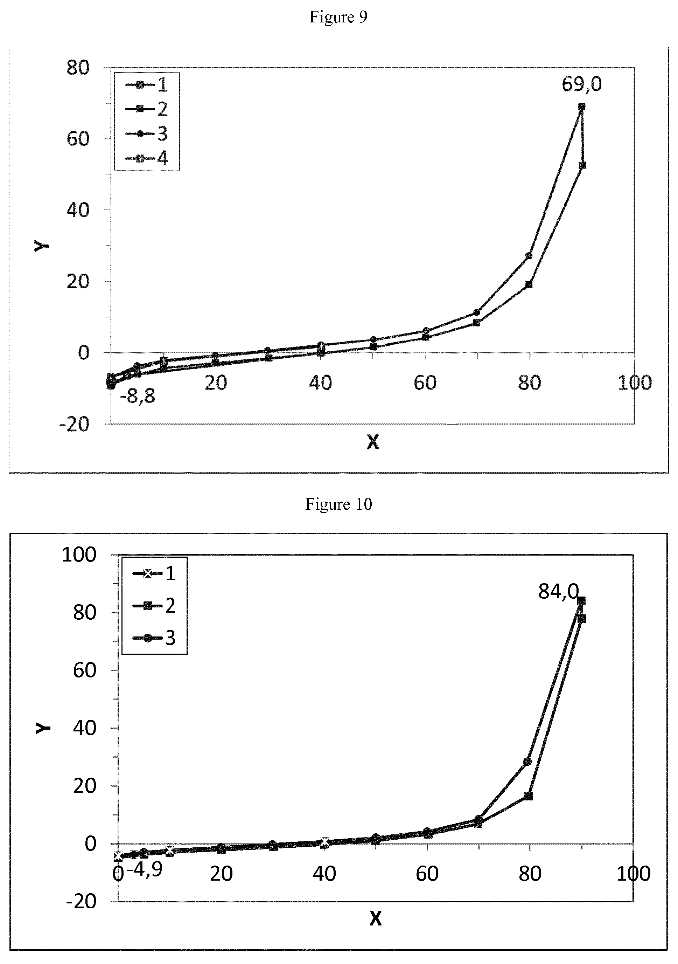

**Figure 9** illustrates a representative DVS isotherm of magnesium aluminometasilicate Neusilin^{®} US2 (used for example in Example 4-1B), recorded as described in Example part. The X-axis shows the relative humidity / % and the Y-axis shows the mass difference Δm / %. The Δm(desorption) values are obtained from the desorption isotherm (symbols: ●), the Δm(adsorption) values are obtained from the adsorption isotherm (symbols: ■).
**Figure 10** illustrates a representative DVS isotherm of micronized synthetic amorphous silica gel Syloid^{®} 72FP (used for example in Example 4-2C), recorded as described in Example part. The X-axis shows the relative humidity / % and the Y-axis shows the mass difference Δm / %. The Δm(desorption) values are obtained from the desorption isotherm (symbols: ●), the Δm(adsorption) values are obtained from the adsorption isotherm (symbols: ■).

### DETAILED DESCRIPTION OF THE INVENTION

In a first embodiment, the present invention relates to a solid dispersion comprising amorphous betrixaban maleate and a pharmaceutically acceptable carrier selected from the group consisting of organic polymers, silicon-based inorganic compounds and mixtures thereof.

Regarding the pharmaceutically acceptable carrier, it was surprisingly found that carriers that exhibit specific characteristics when subjected to a dynamic vapour sorption measurement are especially suitable to stabilize amorphous betrixaban maleate according to the present invention. Regarding the specific characteristics of the preferred pharmaceutically acceptable carrier according to the present invention when subjected to a dynamic vapour sorption measurement, it was found that in the adsorption-desorption isotherms of these pharmaceutically acceptable carrier, the mass difference Δm(desorption) at 75% relative humidity and 25°C is greater than or equal to the mass difference Δm(adsorption) at 75% relative humidity and 25°C. Even more preferably, the mass difference Δm(desorption) at 75% relative humidity and 25°C is greater than the mass difference Δm(adsorption) at 75% relative humidity and 25°C. Without wanting to be bound by any theory, it is believed that the specific pore properties and/or the specific surface properties, either regarding the respective chemical and/or the physical nature thereof, of the preferred pharmaceutically acceptable carrier may lead to their specific and advantageous suitability for stabilizing the amorphous betrixaban maleate in the solid dispersion. The dynamic vapour sorption measurements and the determination of the values of Δm(desorption) and Δm(adsorption) at 75% relative humidity and 25°C are described in the examples of the present invention.

In one aspect, the pharmaceutically acceptable carrier is an organic polymer, preferably a hydrophilic organic polymer, more preferably a water-soluble hydrophilic organic polymer.

Polymers that contain only a single type of repeat unit are known as homopolymers, while polymers containing a mixture of repeat units are known as copolymers. The organic polymer may be a homopolymer or copolymer and is preferably a homopolymer.

With regard to the chemical nature of the organic polymer, polysaccharides and derivatives of polysaccharides are preferred. The polysaccharides can be homoglycans or heteroglycans. Further, the polysaccharides can be naturally occurring compounds or synthesized compounds. Regarding the derivatives of polysaccharides, compounds are preferred which are derivatized at one or more hydroxyl groups of the monosaccharide units of the polysaccharides.

Preferred hydrophilic organic polymers are polysaccharides, preferably cellulose derivatives, polyvinylpyrrolidones, polyethylene glycols, polyethylene glycol based copolymers, polyacrylic acids, salts of polyacrylic acids, polyvinyl alcohols, polyacrylamide copolymers, methacrylic acid copolymers, methacrylate copolymers, pectines, chitin derivatives, chitosan derivatives, polyphosphates, polyoxazolines, polyvinyl caprolactam-polyvinyl acetate-polyethylene glycol copolymers, and mixtures of two or more thereof.

More preferred hydrophilic polymers are cellulose derivatives selected from the group consisting of alkylcellulose, preferably methylcellulose, ethylcellulose, or propylcellulose; hydroxyalkylcellulose, preferably hydroxymethylcellulose, hydroxyethylcellulose, or hydroxypropyl cellulose; hydroxyalkyl alkylcellulose, preferably hydroxyethyl methylcellulose (HEMC), or hydroxypropyl methylcellulose (HPMC); carboxyalkyl cellulose, preferably carboxymethylcellulose (CMC), carboxymethyl hydroxyethylcellulose (CMHEC), hydroxyethyl carboxymethylcellulose (HECMC); sodium carboxymethylcellulose, cellulose acetate phthalate (CAP), hydroxypropyl methylcellulose acetate (HPMCA), hydroxypropyl methylcellulose phthalate (HPMCP), hydroxypropyl methylcellulose acetate succinate (HPMCAS), and a mixture of two or more thereof. Most preferably, the hydrophilic organic polymer comprises, preferably consists of, a cellulose derivative selected from the group consisting of hydroxyalkyl alkylcelluloses and a mixture of two or more thereof. Hydroxypropyl methylcellulose is particularly preferred.

Preferably, the weight average molecular weight (Mw) of the cellulose derivative is in the range of from 7 to 225 kDa, more preferably in the range of from 7 to 100 kDa, most preferably in the range of from 7 to 30 kDa. According to the present invention, it is possible that the solid composition contains two or more cellulose derivatives, preferably two or more hydroxyalkyl alkylcelluloses, more preferably two or more hydroxypropyl methylcelluloses that differ only in the average molecular weight.

The molecular degree of substitution (DS) describes the number of hydroxyalkyl alkylated sites per anhydroglucose unit of a given hydroxyalkyl alkylcellulose. Preferably, the molecular degree of substitution (DS) of the cellulose derivative is in the range of from 0.3 to 2.8, more preferably in the range of from 0.6 to 2.5, more preferably in the range of from 1.0 to 2.3, more preferably in the range of from 1.3 to 2.0. According to the present invention, it is possible that the solid composition contains two or more cellulose derivatives, preferably two or more hydroxyalkyl alkylcelluloses, more preferably two or more hydroxypropyl methylcelluloses that differ only in the molecular degree of substitution.

In another aspect, the pharmaceutically acceptable carrier is a silicon-based inorganic compound, preferably a silicon-based inorganic adsorbent, i.e. a silicon-based inorganic compound having a high porosity and a large surface area that enable it to adsorb high loads of oil or water. Preferred silicon-based inorganic adsorbents are silica, silicates, and a combination of two or more thereof.

In one aspect, the silicon-based inorganic compound is silica. The silica is preferably selected from the group consisting of fumed silica, precipitated silica, gel silica, colloidal silica, and a combination of two or more thereof, such as a combination of fumed silica and precipitated silica or a combination of fumed silica and colloidal silica or a combination of fumed silica and gel silica or a combination of precipitated silica and gel silica or a combination of precipitated silica and colloidal silica or a combination of gel silica and colloidal silica or a combination of fumed silica and precipitated silica and gel silica or a combination or fumed silica and gel silica and colloidal silica or a combination of precipitated silica and gel silica and colloidal silica or a combination of fumed silica and precipitated silica and gel silica and colloidal silica. Preferred silica include, but are not restricted to, the commercially available compounds Syloid® 72 FP, Syloid® 244 FP, both from Grace.

In another aspect, the silicon-based compound is a silicate. The silicate is preferably an aluminosilicate or an aluminometasilicate which, more preferably, additionally contains at least one alkali metal element selected from the group consisting of Li, Na, K, Rb, Cs and a combination of two or more thereof, preferably from the group consisting of Li, Na, K, and a combination of two or more thereof, more preferably from the group consisting of Na, K, and a combination of two or more thereof, and/or at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminosilicate or an aluminometasilicate which additionally contains at least one alkaline earth metal element selected from the group consisting of Mg, Ca, Sr, Ba, and a combination of two or more thereof, preferably from the group consisting of Mg, Ca, Ba, and a combination of two or more thereof, preferably from the group consisting of Mg, Ca, and a combination of two or more thereof. More preferably, the silicate is an aluminometasilicate, which additionally contains Mg. Preferred magnesium aluminometasilicate include, but are not restricted to, the commercially available compounds Neusilin® UFL2, Neusilin® US2, both from Fuji Chemical Industry Co., Ltd.

Examples of silicates include, but are not restricted to, nesosilicates comprising the structure unit [SiO_{4]4}-, sorosilicates comprising the structure unit [Si₂O₇]₆-, cyclosilicates comprising the structure unit [SiₙO₃ₙ]₂ₙ-, single chain inosilicates comprising the structure unit [SiₙO₃ₙ]₂ₙ-, double chain inosilicates comprising the structure unit [Si₄ₙO11ₙ]₆ₙ-, phyllosilicates comprising the structure unit [SiₙO₅ₙ]₂ₙ-, or tectosilicates with a 3D framework comprising the structure unit [AlₓSi_{y}O_{2(x+y)}]ₓ-.

Silicon-based inorganic compounds have preferably a pH in a defined range, preferably a pH of at least 6.0 as determined using a pH meter in a solution of 400 mg of the silicon-based inorganic compound in 10 mL of de-ionized water at room temperature. More preferably, the at least one silicon-based compound has a pH in the range of from 6.0 to 9.0, more preferably in the range of from 6.3 to 8.5, more preferably in the range of from 6.6 to 8.0.

Generally, it is conceivable that the solid dispersion of the present invention contains at least one silicon-based inorganic compound having a pH in the above-defined preferred ranges and at least one silicon-based inorganic compound having a pH outside these ranges. Preferably, all silicon-based inorganic compounds comprised in the solid composition of the present invention have a pH in the above-defined preferred ranges.

Preferably, the bulk density of silicon-based inorganic compound is in the range of from 10 to 500 g/ml, preferably in the range of from 30 to 400 g/ml, more preferably in the range of from 50 to 300 g/ml. Generally, it is conceivable that the solid dispersion of the present invention contains at least one silicon-based inorganic compound having a bulk density in the above-defined preferred ranges and at least one silicon-based inorganic compound having a bulk density outside these ranges. Preferably, all silicon-based inorganic compound comprised in the solid composition of the present invention have a bulk density in the above-defined preferred ranges.

Generally, the silica and/or the silicate can be present in crystalline or amorphous form. Preferably, at least 90 weight-%, more preferably at least 95 weight-%, more preferably at least 99 weight-% of the at least one silicon-based inorganic compound are present in amorphous form. More preferably, at least 99.5 weight-%, more preferably at least 99.9 weight-%, more preferably at least 99.99 weight-% of the at least one silicon-based inorganic compound are present in amorphous form.

The solid dispersion according to the present invention can also comprise a mixture of an organic polymer and a silicon-based inorganic compound.

The weight ratio of betrixaban maleate and the pharmaceutically acceptable carrier is preferably from 1:1 to 1:10, more preferably from 1:1.5 to 1:8, even more preferably from 1:2 to 1:5. The weight ratio is calculated based on the total amount of betrixaban maleate present in said solid dispersion and based on the total amount of the pharmaceutically acceptable carriers present in said solid dispersion, i.e. if two or more pharmaceutically acceptable carriers are present in the solid dispersion of the present invention, the combined weight of these is taken for the calculation of the weight ratio.

In a preferred embodiment, the composition of the invention comprises betrixaban maleate in substantially amorphous form. In other words, the composition of the invention comprises at most 20%, preferably at most 10%, more preferably at most 5%, even more preferably at most 2% and most preferably at most 1% by weight of any crystalline form of betrixaban maleate, in particular of any one of the known Forms I, II and III as defined above, as judged by the absence of their characteristic peaks.

The absence of peaks in the powder X-ray diffractogram of the composition of the invention, in particularly the absence of characteristic PXRD peaks of Forms I, II and III of betrixaban maleate as defined above, shows that betrixaban maleate is substantially in amorphous form. This is evidenced by Figures 1, 3, 4 and 5.

In a preferred embodiment, the composition of the invention comprises betrixaban maleate that is substantially in amorphous form after storage under normal conditions, preferably after storage at a relative humidity of 45% at 23°C for at least six months, more preferably for at least one year, most preferably for at least two years. Alternatively or additionally, the composition of the invention comprises betrixaban maleate that is substantially in amorphous form after storage under accelerated conditions, preferably after storage at a relative humidity of 70 % at 40°C for at least one month, more preferably for at least three months, most preferably for at least six months. In a particularly preferred embodiment, the amorphous betrixaban maleate comprised in the composition of the invention is stable in the amorphous physical form after storage under normal and accelerated conditions.

In a further preferred embodiment, the solid dispersion of the present invention is a solid dispersion wherein amorphous betrixaban maleate is chemically stable. By "chemically stable" it is meant that amorphous betrixaban maleate present in the solid dispersion of the present invention shows very little degradation upon storage under normal or accelerated conditions. Very little degradation means that an HPLC analysis of betrixaban maleate shows no impurity of more than 0.1 area%.

### Dichloromethane solvate of betrixaban maleate

The present invention also refers to a crystalline solvate of betrixaban maleate, preferably to crystalline dichloromethane solvate of betrixaban maleate (see Example 2) and to a process for its preparation. As it will be explained below, said solvate can be useful as an intermediate for the preparation of the solid dispersion of the invention. The dichloromethane solvate of betrixaban maleate may be characterized by a stoichiometric composition, i.e. number of solvent molecules per betrixaban maleate molecule, of 0.8 ± 0.1 mol of dichloromethane.

Thus, in one embodiment, the present invention refers to dichloromethane solvate of betrixaban maleate, having a characteristic powder X-ray diffractogram (PXRD) pattern with reflections at 2-theta values of 4.5 ± 0.2, 9.1 ± 0.2,12.5 ± 0.2, 16.8 ± 0.2,21.9 ± 0.2 and 26.1 ± 0.2°, when measured at a temperature in the range of from 15 to 25°C with Cu-Kalpha1,2 radiation having a wavelength of 0.15419 nm. Thermogravimetric analysis (TGA) of dichloromethane solvate of betrixaban maleate indicates a total weight loss between 35°C and 135°C of ca. 11.0% (and 0.5% between 135°C and 190°C; 1.0 mole equivalent of dichloromethane corresponds to ca. 13.0%).

The present invention also relates to a process for the preparation of crystalline dichloromethane solvate of betrixaban maleate comprising the steps of
(i) providing a solution of betrixaban maleate in dichloromethane,
(ii) optionally adding seed crystals of the dichloromethane solvate of betrixaban maleate
(iii) crystallizing the dichloromethane solvate of betrixaban maleate
(iv) optionally isolating the crystalline solvate in solid form, and
(v) optionally drying the crystalline solvate.

Optionally, seed crystals may be added in step (ii). The seed crystals are prepared with the same solvent as the one used in step (i) of the process. In particular, the seed crystals are prepared using the same process steps as the solvate of the present invention. The seed crystals are typically added in an amount of 0.1 wt% to 10 wt%, preferably in an amount of 0.5 wt% to 7.0 wt%, most preferably 1.0 wt.% 5.0 wt%, on the basis of the total amount of the starting material used in step (i).

In step (iii) the solution is stirred at a temperature in the range of from 15 to 30°C, preferably in the range of from 20 to 30°C and kept at this temperature, typically for a period of time from 2 to 24 hours, under stirring conditions or without stirring, in order to promote crystallization.

Optionally, isolation in step (iv) may be performed by using procedures known in the art, such as by filtration, centrifugation, or evaporation of solvent. Moreover, the isolated crystals may optionally be dried in step (v), e.g. under reduced pressure, typically at room temperature, or heated up to a temperature between 25°C and 40°C or the crystals may directly be used in further processes, such as the preparation of amorphous solid dispersion of betrixaban maleate or isolated crystals may be used as seed crystals for the preparation of a solvate of betrixaban maleate.

### Preparation process of the solid dispersion

The present invention also relates to a process for preparing the solid dispersion of the invention comprising the steps of
a) providing betrixaban maleate;
b) dissolving or dispersing betrixaban maleate and the pharmaceutically acceptable carrier in a solvent to form a mixture; and
c) removing the solvent to provide the solid dispersion.

The present invention also relates to a solid dispersion obtained or obtainable by the above-described process.

Any form of betrixaban maleate may be used, such as crystalline, amorphous form, or a mixture of thereof. Betrixaban maleate may be present in crystalline Form I or Form II or Form III or as a mixture of two or more thereof. Alternatively, betrixaban maleate in step (a) may be provided in amorphous form, for example according to Comparative Example 1 of the present invention. Betrixaban maleate may also be provided as a crystalline solvate with a solvation partner, which is a chlorohydrocarbon, preferably dichloromethane.

In step (b), betrixaban maleate and the pharmaceutically acceptable carrier are dissolved or dispersed in a suitable solvent. Both components may be dissolved or dispersed together or subsequently.

The term "a suitable solvent" as used herein refers to a solvent or solvent mixture in which both betrixaban maleate and the pharmaceutically acceptable carrier have adequate solubility or may be suitably dispersed. The term "adequate solubility" means a solubility at room temperature of greater than about 10 mg/ml.

Preferred suitable solvents are water, acetonitrile, C1-C3 ketones, C1-C2 halogenated hydrocarbons, C3-C4 alcohols, C2-C6 ethers, C3-C5 esters, and a combination of two or more thereof. More preferably, the solvent comprises, and for example consists of, dichloromethane, chloroform, ethanol, methanol, THF, methyl THF, 2-propanol, ethyl acetate, acetone, acetonitrile, water or mixtures of two or more thereof. Even more preferably, the solvent comprises, and for example consists of, dichloromethane, THF, methyl THF, acetone, acetonitrile, water or mixtures of two or more thereof. More preferably, the solvent comprises dichloromethane, acetone, acetonitrile, water or mixtures of two or more thereof. Most preferably the solvent comprises, more preferably the solvent is acetonitrile, water or mixtures of acetonitrile and water.

To accelerate and/or improve the dissolution process of betrixaban maleate in the solvent, suitable methods can be applied. For example, the dissolution process can be influenced by choosing suitable temperatures, by stirring, and/or by subjecting the respective mixtures to sonication, wherein these methods can be applied during the entire or one or more parts of the mixing process.

Preferably, the weight ratio of betrixaban maleate plus the pharmaceutically acceptable carrier relative to the solvent is in the range of from 0.01:1 to 0.4:1, more preferably in the range of from 0.01:1 to 0.2:1.

In the case where the pharmaceutically acceptable carrier is an organic polymer, betrixaban maleate and the organic polymer may require different solvents to obtain the desired solubility. A mixture of solvents can then be used. In this case, betrixaban maleate may be dissolved in a solvent to give a mixture comprising the solvent and betrixaban maleate. Likewise, the organic polymer may be dissolved in a further solvent to give a mixture comprising the polymer and the further solvent. Both mixtures may then be mixed together. Preferably, the solution of betrixaban maleate and the organic polymer in the solvent is prepared at a temperature in the range of from 10 to 40°C, more preferably in the range of from 20 to 30°C, preferably at ambient pressure.

In the case where the pharmaceutically acceptable carrier is a silicon-based inorganic compound, it is preferred that the process comprises dispersing the silicon-based inorganic compound in a solution comprising the dissolved betrixaban maleate. Consequently, solvents are preferred in which betrixaban maleate can be dissolved and the silicon-based inorganic compound can be dispersed. Preferably, the dispersion of the silicon-based inorganic compound, is prepared at a temperature in the range of from 10 to 40°C, more preferably in the range of from 20 to 30°C, preferably at ambient pressure.

Regarding the weight ratio of betrixaban maleate and the silicon-based inorganic compound relative to the solvent, no specific restrictions exist provided that the finally obtained mixture is a mixture wherein the silicon-based inorganic compound is dispersed in a solution of the betrixaban maleate in the solvent, which mixture can be subjected to the subsequent step (c).

The solution obtained in step (b) may be directly used in step (c) of the method according to the invention. According to a preferred embodiment, the solution formed is purified before used in step (b). The term "purified" in this context means that non-dissolved particles, such as non-dissolved organic polymer and/or non-dissolved betrixaban maleate, may be removed by suitable methods known to those skilled in the art such as centrifugation, filtration, ultrafiltration or the like. Preferably, the solution in step (b) is filtrated prior to step (c).

In step (c) of the above-described process, at least part, preferably essentially all, of the solvent is removed. "Essentially all" means that at least 95 % by weight, more preferably at least 99 % by weight, more preferably at least 99.9 % by weight of the solvent present in the mixture according to step (b) is removed in step (c).

Preferably, the solid dispersion obtained or obtainable by this process thus comprises less than 5 % by weight, more preferably less than 1 % by weight, more preferably less than 0.1 % by weight, based on the total weight of the solid dispersion, of the solvent. Most preferably, all of the solvent present in the solution is removed to give the solid dispersion.

The removal of the solvent may be carried out by any suitable method known to those skilled in the art such as evaporation, spray drying, lyophilization, melt extrusion, drum drying, freeze-drying or other solvent removal processes. Preferably, the solvent is removed by freeze drying such as lyophilization, by spray drying or by vacuum drying or evaporation. Spray drying is a process well known to those skilled in the art for preparing solid dispersions. In such a spray drying process, the solution is pumped through an atomizer into a drying chamber thereby removing the solvent to form the solid dispersion. A drying process uses hot gases, such as air, nitrogen, nitrogen-enriched air or argon, to dry the particles. The solution can be atomized by conventional means well known in the art, such as a two-fluid sonication nozzle and a two-fluid non-sonication nozzle. Preferably, the solvent is removed by lyophilization or spray drying.

If the solvent is removed by lyophilization, the sample temperature during lyophilization may be varied or held essentially constant and is preferably in the range of from 20 to 40°C, more preferably in the range of from 25 to 40°C.

In a further aspect of the present invention, step a) of the above preparation process further comprises the initial steps of
(A1) providing a solution of betrixaban in a suitable solvent and adding maleic acid,
(A2) optionally concentrating the obtained mixture.

The solvent used in step A1) may be the same or different from the solvent used in step b) above. In a particularly preferred embodiment, the same solved is used.

### Preparation process of amorphous betrixaban maleate

The present invention also relates to a process for the preparation of amorphous betrixaban maleate, the process comprising the steps of
(a') providing betrixaban maleate;
(b') dissolving betrixaban maleate provided in (a') in a suitable solvent; and
(c') removing essentially all of the solvent.

In step (a') any form of betrixaban maleate may be used, such as crystalline, amorphous form, or a mixture of thereof. Betrixaban maleate may be present in crystalline Form I or Form II or Form III or as a mixture of two or more thereof. Betrixaban maleate may also be provided as a crystalline solvate with a solvation partner, which is a chlorohydrocarbon, preferably dichloromethane.

Betrixaban maleate may also be provided in solution according to steps (A1) to (A2) of the present invention.

In step (b') of the above described method, examples of suitable solvents include, but are not limited to, water, acetonitrile, C1-C3 ketones, C1-C2 halogenated hydrocarbons, C3-C4 alcohols, C2-C6 ethers, C3-C5 esters, and a combination of two or more thereof. More preferably, the solvent comprises, and for example consists of, dichloromethane, chloroform, ethanol, methanol, THF, methyl THF, 2-propanol, ethyl acetate, acetone, acetonitrile, water or mixtures of two or more thereof. Preferably, the solvent comprises, and for example consists of, dichloromethane, THF, methyl THF, acetone, acetonitrile, water or mixtures of two or more thereof. More preferably, the solvent comprises, and for example consists of, acetonitrile, water or mixtures thereof.

The solution obtained in step (b') may be directly used in step (c') of the method according to the invention. According to a preferred embodiment, the solution formed in (b') is purified before used in step (c'). Preferably, the solution in step (b') is filtrated prior to step (c').

### Step (c')

In step (c') of the above described method, at least part, preferably essentially all, of the solvent is removed.

The removal of the solvent may be carried out by any suitable method known to those skilled in the art such as evaporation, spray drying, lyophilization, melt extrusion, drum drying, freeze-drying or other solvent removal processes. Preferably, the solvent is removed by freeze drying such as lyophilization, by spray drying or by vacuum drying or evaporation, more preferably by lyophilization or spray drying.

If the solvent is removed by lyophilization, the sample temperature during lyophilization may be varied or held essentially constant and is preferably in the range of from 20 to 40°C, more preferably in the range of from 25 to 40°C.

### Pharmaceutical compositions and use

In a further embodiment, the present invention relates to a pharmaceutical composition comprising the solid dispersion of the invention and one or more pharmaceutically acceptable excipients.

The one or more pharmaceutically acceptable excipient(s) is/are preferably selected from the group consisting of carriers, fillers, diluents, lubricants, glidants, sweeteners, stabilizing agents, solubilizing agents, antioxidants and preservatives, flavouring agents, binders, colorants, osmotic agents, buffers, surfactants, disintegrants, granulating agents, coating materials and combinations thereof. In a preferred embodiment, the one or more pharmaceutically acceptable excipient(s) is/are selected from the group consisting of diluents, binders, disintegrants, lubricants and glidants.

The pharmaceutical composition of the present invention is preferably an oral solid dosage form, such as a tablet or a capsule. Preferably, the pharmaceutical composition of the present invention is a hard gelatine capsule.

Preferably, the pharmaceutical composition comprises an amount of betrixaban maleate of 40, 60 or 80 mg, calculated as betrixaban free base.

The pharmaceutical composition of the present invention can be prepared by wet or dry processing methods. In certain embodiments the pharmaceutical composition is prepared by wet processing methods, such as, but not limited to, wet granulation methods. Suitable wet granulation methods comprise high-shear granulation or fluid-bed granulation. In another embodiment the pharmaceutical composition is prepared by dry processing methods, such as, but not limited to, direct compression or dry granulation methods. An example of dry granulation is roller compaction. The pharmaceutical composition obtained by dry or wet processing methods may be compressed into tablets, encapsulated or metered into sachets. Preferably, the pharmaceutical composition is encapsulated.

In a further aspect, the present invention relates to amorphous betrixaban maleate or the pharmaceutical composition comprising the same as described above for use as a medicament.

In yet another aspect, the present invention relates to amorphous betrixaban maleate or the pharmaceutical composition comprising the same as described above for use in the prevention and the treatment of disease conditions characterized by undesired thrombosis.

### Advantages

The present invention circumvents the drawbacks of pharmaceutical compositions comprising the known crystalline forms of betrixaban maleate, i.e. Forms I, II and III. The solid dispersion of the present invention can be prepared in a reliable and reproducible way and retains its amorphous character upon storage, for example throughout its shelf life, as well as over the whole range of relative humidity.

The betrixaban maleate in the context of the solid dispersion of the present invention is stable in the amorphous state upon storage, as judged by the absence of characteristic PXRD peaks after storage. Amorphous betrixaban maleate readily converts to crystalline betrixaban maleate Form I, as described above, upon storage for as short as one week only if not formulated in the solid dispersion of the invention.

### EXAMPLES

### Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta. Thus, a diffraction peak that appears for example at 9.0 ° 2-theta can appear between 8.8 and 9.2 ° 2-theta on most X-ray diffractometers under standard conditions.

### Differential scanning calorimetry

DSC was performed on a Mettler Polymer DSC R instrument. The sample was heated in a 40-microL aluminium pan with pierced aluminium lid from 25 to 250°C at a rate of 10 K/min. Nitrogen (purge rate 50 mL/min) was used as purge gas.

### Thermogravimetric analysis

TGA was performed on a Mettler TGA/DSC 1 instrument. The sample was heated in a 100 micro aluminium pan closed with an aluminium lid. The lid was automatically pierced at the beginning of the measurement. The sample was heated from 25 to 250°C at a rate of 10 K/min. Nitrogen (purge rate 50 mL/min) was used as purge gas.

### Dynamic Vapour Sorption (DVS) measurements - Determination of Δm(desorption) and Δm(adsorption) at 75% relative humidity and 25°C

The adsorption-desorption isotherms from which the values of Δm(desorption) and Δm(adsorption) at 75 % relative humidity and at 25°C were obtained, were recorded with an SPSx-1µ (1 micro) moisture sorption analyser (ProUmid GmbH & Co. KG, Ulm, Germany).

A given measurement cycle was started at ambient relative humidity (RH), in the present case 40% RH. The RH was decreased to 3% and then to 0%. For this isotherm, as black filled square with a white x inside is used in the respective Figures. Subsequently, the adsorption isotherm was recorded, i.e. RH was increased to 5%, then to 10%, and thereafter in 10% steps. Once having reached the chosen maximum RH value, the desorption isotherm was recorded, starting with 10% steps down to a RH of 10%, followed by a RH decrease in 5% steps to 0% RH. The last step consisted of increasing the RH to ambient RH. As to the isotherm obtained by the last step, a black filled square with a white star inside is used as symbol in the respective Figures.

The time per step was set to 3 to 5 hours. For all steps and all isotherms, the temperature was set to 25 ± 0.1°C.

To obtain the Δm(desorption) and Δm(adsorption) values, the recorded adsorption-desorption isotherms shown in the Figures 7 to 10 of the present invention were analysed by comparing the value of Δm(desorption), plotted on the Y-axis, of a given desorption isotherm with the value of Δm(adsorption), plotted on the Y-axis, of the respective adsorption isotherm, both at 75% RH, plotted on the X-axis.

### Determination of the moisture stability

Moisture stability at accelerated stability conditions was performed in a Memmert constant climate chamber HPP110. 50-70 mg of a given solid dispersion were exposed to an atmosphere having a relative humidity of 75±1 % and a temperature of 40±0.5°C for a period of time as indicated in Tables 1 and 4-7 below and analysed via PXRD as described above.

### Determination of the pH of the silicon-based inorganic adsorbent

To 400 mg of a given silicon-based inorganic adsorbent were added 10 mL of de-ionized water at room temperature. After stirring for 2 minutes, the mixture was allowed to stand for 2 minutes at room temperature. The pH of the aqueous phase was then determined using pH meter.

### Comparative Example 1: Physical stability of amorphous betrixaban maleate under accelerated stability conditions

Betrixaban maleate (3.00 g, e.g. prepared according to the procedure disclosed in WO 2011/084519, Example 2) was dissolved in 100 mL of acetonitrile/water solution (volume ratio 50:50). The solution was filtered. The homogeneous solution was frozen in a bath of liquid nitrogen and lyophilized at a pressure of from 0 to 2 mbar, yielding amorphous betrixaban maleate, as confirmed by the PXRD depicted in Figure 1. The obtained amorphous betrixaban maleate was open stored under accelerated stability conditions (temperature: 40°C, relative humidity: 75%). The material was analysed by powder X-ray diffraction after one, three, and six weeks and the results are summarized in Table 1.

**Table 1: Accelerated stability test of neat amorphous betrixaban maleate**

| **Sample** | **Carrier** | **Physical form** | **PXRD analysis after storage at 40°C and 75% RH for** | | |
|---|---|---|---|---|---|
| | | | **1 week** | **3 weeks** | **6 weeks** |
| Betrixaban maleate | - | amorphous | Form I | Form I | Form I |

As can be seen from Table 1, neat amorphous betrixaban maleate is physically unstable and readily crystallizes during the storage under accelerated stability conditions. After one week upon storage at a temperature of 40°C and a relative humidity of 75% sample contained crystalline Form I of betrixaban maleate. PXRD pattern of the betrixaban maleate sample after one week upon storage at a temperature of 40°C and a relative humidity of 75% is shown in Figure 2.

Neat amorphous betrixaban maleate was also subjected to a dynamic vapour sorption (DVS) experiment until 95% relative humidity as described above. During the DVS experiment betrixaban maleate crystalized. The PXRD pattern of the sample after the DVS experiment was essentially identical to the one shown in Figure 2.

### Example 1: Preparation of amorphous solid dispersions comprising betrixaban maleate and Neusilin^{®} UFL2

### Example 1-1

To betrixaban maleate (98.5 mg, prepared according to the procedure disclosed in WO 2011/084519, Example 2) and magnesium aluminometasilicate (Neusilin^{®} UFL2 from Fuji Chemical Industry Col., Ltd., 98.4 mg) were added 8 mL of acetonitrile/water solution (volume ratio 50:50), leading to a solution of betrixaban maleate with suspended Neusilin^{®} UFL2. The suspension was homogeneously frozen in a bath of liquid nitrogen and lyophilized at a pressure of from 0 to 2 mbar, yielding amorphous solid dispersions of betrixaban maleate. The resulting solid dispersion was subjected to a moisture stability test as described above. The results are summarized in Table 5 below. Representative PXRD pattern of the solid dispersion after its preparation is shown in Figure 3, the PXRD pattern of the solid dispersion after six weeks upon storage at a temperature of 40°C and a relative humidity of 75% is shown in Figure 4. Figures 3 and 4 show that the betrixaban maleate comprised in the solid dispersion was in amorphous form after its preparation and that Neusilin^{®} UFL2 could stabilize amorphous betrixaban maleate during the moisture stability test.

The amorphous solid dispersion of betrixaban maleate was also subjected to a dynamic vapour sorption (DVS) experiment until 95% relative humidity as described above. During the DVS experiment the solid dispersion remained amorphous. The PXRD pattern of the sample after the DVS experiment was essentially identical to the one shown in Figure 5.

### Example 1-2

To betrixaban maleate (35.9 mg, prepared according to the procedure disclosed in WO 2011/084519, Example 2) and Neusilin® UFL2 (72.0 mg) were added 8 mL of acetonitrile/water solution (volume ratio 50:50), leading to a solution of betrixaban maleate with suspended Neusilin® UFL2. The suspension was homogeneously frozen in a bath of liquid nitrogen and lyophilized at a pressure of from 0 to 2 mbar, yielding amorphous solid dispersions of betrixaban maleate. The resulting solid dispersion was subjected to a moisture stability test as described above. The results are summarized in Table 6 below. The PXRD pattern of the solid dispersion after its preparation was essentially identical to the one shown in Figure 3, the PXRD pattern of the solid dispersion after six weeks upon storage at a temperature of 40°C and a relative humidity of 75% was essentially identical to the one shown in Figure 5. Betrixaban maleate comprised in the solid dispersion was in amorphous form and did not crystallize during the moisture stability test.

The amorphous solid dispersion of betrixaban maleate was also subjected to a dynamic vapour sorption (DVS) experiment until 95% relative humidity as described above. During the DVS experiment the solid dispersion remained amorphous. The PXRD pattern of the sample after the DVS experiment was essentially identical to the one shown in Figure 5.

### Example 1-3:

To betrixaban maleate (34.5 mg, prepared according to the procedure disclosed in WO2011/084519, Example 2) and Neusilin® UFL2 (103.6 mg) were added 8 mL of acetonitrile/water solution (volume ratio 50:50), leading to a solution of betrixaban maleate with suspended Neusilin® UFL2. The suspension was homogeneously frozen in a bath of liquid nitrogen and lyophilized at a pressure of from 0 to 2 mbar, yielding amorphous solid dispersions of betrixaban maleate. The resulting solid dispersion was subjected to a moisture stability test as described above. The results are summarized in Table 7 below. The PXRD pattern of the solid dispersion after its preparation was essentially identical to the one shown in Figure 3, the PXRD pattern of the solid dispersion after six weeks upon storage at a temperature of 40°C and a relative humidity of 75% is shown in Figure 5. Figure 5 shows that the betrixaban maleate comprised in the solid dispersion did not crystallize during the moisture stability test.

The amorphous solid dispersion of betrixaban maleate was also subjected to a dynamic vapour sorption (DVS) experiment until 95% relative humidity as described above. During the DVS experiment the solid dispersion remained amorphous. The PXRD pattern of the sample after the DVS experiment was essentially identical to the one shown in Figure 5.

### Example 1-4

To dichloromethane solvate of betrixaban maleate (60.1 mg, prepared according to Example 2) and Neusilin® UFL2 (53.4 mg) were added 8 mL of acetonitrile/water solution (volume ratio 50:50), leading to a solution containing betrixaban maleate with suspended Neusilin^{®} UFL2. The suspension was homogeneously frozen in a bath of liquid nitrogen and lyophilized at a pressure of from 0 to 2 mbar, yielding amorphous solid dispersions of betrixaban maleate. The PXRD pattern of the solid dispersion after its preparation was essentially identical to the one shown in Figure 3.

### Example 1-5

Crystalline betrixaban (40.0 mg, prepared according to the procedure disclosed in WO2001/19788, Example 266) and maleic acid (10.3 mg) were dissolved in 2 mL of acetonitrile/water solution (volume ratio 50:50). The solution was stirred at room temperature for 5 minutes. Neusilin® UFL2 (53.4 mg) and 6 mL of acetonitrile/water solution (volume ratio 50:50) were added to the solution, leading to a mixture containing a solution of betrixaban maleate with suspended Neusilin® UFL2. The suspension was homogeneously frozen in a bath of liquid nitrogen and lyophilized at a pressure of from 0 to 2 mbar, yielding amorphous solid dispersions of betrixaban maleate. The PXRD pattern of the solid dispersion after its preparation was essentially identical to the one shown in Figure 3.

### Example 2: Preparation of crystalline betrixaban maleate dichloromethane solvate

To amorphous betrixaban maleate (200 mg, for example prepared according to the procedure described in Comparative Example 1) were added 10.0 mL of dichloromethane at room temperature under stirring conditions, leading to a suspension formation. The suspension was stirred at room temperature for one hour. The solid material was collected by filtration and dried at room temperature under vacuum (about 30 mbar) for 18 hours to yield 201 mg of betrixaban maleate dichloromethane solvate. Characteristic PXRD pattern of the obtained betrixaban maleate dichloromethane solvate is shown in Figure 6. The corresponding peak list is provided in Table 2 below (relative peak intensities can vary due to e.g. inter-apparatus variability, sample crystallinity, sample preparation, etc.).

**Table 2: peak list of the PXRD pattern of betrixaban maleate dichloromethane solvate**

| **position / ° 2-theta** | **relative intensity /%** | | **position / ° 2-theta** | **relative intensity /%** |
|---|---|---|---|---|
| 4.5 ± 0.2 | 26 | | 21.2 ± 0.2 | 21 |
| 9.1 ±0.2 | 23 | | 21.9 ± 0.2 | 58 |
| 12.5 ± 0.2 | 30 | | 22.9 ± 0.2 | 28 |
| 13.0 ± 0.2 | 17 | | 23.3 ± 0.2 | 24 |
| 14.1 ± 0.2 | 24 | | 24.1 ± 0.2 | 10 |
| 14.8 ± 0.2 | 15 | | 25.3 ± 0.2 | 36 |
| 16.3 ± 0.2 | 18 | | 25.6 ± 0.2 | 89 |
| 16.8 ± 0.2 | 34 | | 25.9 ± 0.2 | 87 |
| 17.1 ± 0.2 | 20 | | 26.1 ± 0.2 | 100 |
| 17.9 ± 0.2 | 13 | | 26.5 ± 0.2 | 27 |
| 18.4 ± 0.2 | 47 | | 27.0 ± 0.2 | 23 |
| 20.1 ± 0.2 | 21 | | 27.4 ± 0.2 | 10 |
| 20.3 ± 0.2 | 21 | | 28.2 ± 0.2 | 11 |
| 20.9 ± 0.2 | 16 | | | |

DSC curve of the obtained betrixaban maleate dichloromethane solvate shows a first endotherm with an onset temperature of about 90°C (peak maximum at about 126°C), corresponding to sample desolvation. Further heating leads to sample conversion to crystalline Form I of betrixaban maleate. Melting endotherm of Form I is then observed with an onset temperature of about 194°C (peak maximum at about 195°C).

TGA curve of the obtained betrixaban maleate dichloromethane solvate indicates a total weight loss between 35°C and 190°C of ca. 11.5% (1.0 mole equivalent of dichloromethane corresponds to ca. 13.0%).

### Example 3: Preparation of solid dispersions with amorphous betrixaban maleate and

### organic polymers

### Example 3-1: weight ratio betrixaban maleate / organic polymer 1:1

Betrixaban maleate (about 85 mg, e.g. prepared according to the procedure disclosed in WO 2011/084519, Example 2) and the organic polymer (about 85 mg) according to Table 3 were dissolved in 7 to 8 mL of acetonitrile/water solution (volume ratio 50:50). The homogeneous solution was frozen in a bath of liquid nitrogen and lyophilized at a pressure of from 0 to 2 mbar, yielding amorphous solid dispersions of betrixaban maleate. The samples were analysed by powder X-ray diffraction and the results are summarized in Table 3.

**Table 3: Solid dispersions of amorphous betrixaban maleate and polymer carrier (weight ratio API / organic polymer 1:1)**

| **Example** | **Carrier** | | **Physical form** |
|---|---|---|---|
| | **Compound** | ΔΔm (%) | |
| A | HPMC | ≥ 0 | amorphous |
| B | HPC | < 0 | amorphous |
| C | PVP 40 | < 0 | amorphous |
| D | Soluplus^{®} | < 0 | amorphous |
| E | Kollidon^{®} VA64 | < 0 | amorphous |

ΔΔm is the difference between the mass difference Δm(desorption) at 75 % relative humidity and 25°C and the mass difference Δm(adsorption) at 75 % relative humidity and 25°C. HPMC: hydroxypropyl methylcellulose (Methocel E5); HPC: hydroxypropylcellulose (Klucel LF); PVP 40: Polyvinylpyrrolidone (average Mw = 40000 g/mol); Soluplus^{®}: a polyvinylcaprolactam-polyvinylacetate-polyethyleneglycol copolymer commercialized by BASF SE; Kollidon^{®} VA64: a vinyl pyrrolidone-vinyl acetate copolymer (copovidon) commercialized by BASF SE.

### Example 3-2: weight ratio betrixaban maleate / organic polymer 1:3

Betrixaban maleate (about 25 mg, e.g. prepared according to the procedure disclosed in WO 2011/084519, Example 2) and hydroxypropyl methylcellulose (HPMC) (about 75 mg) were dissolved in 8 mL of acetonitrile/water solution (volume ratio 50:50). The homogeneous solution was frozen in a bath of liquid nitrogen and lyophilized at a pressure of from 0 to 2 mbar, yielding an amorphous solid dispersion of betrixaban maleate. The so obtained solid dispersion was open stored under accelerated stability conditions (temperature: 40°C, relative humidity: 75%) for six weeks. The sample was periodically analysed by powder X-ray diffraction and the results are summarized in Table 4.

**Table 4: Accelerated stability test of solid dispersions of amorphous betrixaban maleate and HPMC (weight ratio API/ organic polymer 1:3)**

| **Sample** | **Carrier** | **Physical form** | **PXRD analysis after storage at 40°C and 75% RH for** | | |
|---|---|---|---|---|---|
| | | | **1 week** | **3 weeks** | **6 weeks** |
| A | HPMC | amorphous | amorphous | amorphous | amorphous |

| | | | | | |
|---|---|---|---|---|---|
| HPMC: hydroxypropyl methylcellulose (Methocel E5). | | | | | |

### Example 4: Preparation of solid dispersions with amorphous betrixaban maleate and various silicon-based compounds and physical stability upon storage under accelerated conditions

### Example 4-1: weight ratio betrixaban maleate / silicon-based compound 1:1

To betrixaban maleate (about 100 mg, prepared according to the procedure disclosed in WO 2011/084519, Example 2) and silicon-based compound (about 100 mg) according to Table 5 were added 7-8 mL of acetonitrile/water solution (volume ratio 50:50), leading to a solution of betrixaban maleate with suspended silicon-based compound. The suspension was homogeneously frozen in a bath of liquid nitrogen and lyophilized at a pressure of from 0 to 2 mbar, yielding amorphous solid dispersions of betrixaban maleate. The so obtained solid dispersions were open stored under accelerated stability conditions (temperature: 40°C, relative humidity: 75%) for six weeks. The samples were periodically analysed by powder X-ray diffraction and the results are summarized in Table 5.

**Table 5: Accelerated stability test of solid dispersions of amorphous betrixaban maleate and silicon-based compound (weight ratio API / silicon-based compound 1:1)**

| **Example** | **Carrier** | | **Physical form** | **PXRD analysis after storage at 40°C and 75% RH for** | | |
|---|---|---|---|---|---|---|
| | **Compound** | ΔΔm (%) | | **1 week** | **3 weeks** | **6 weeks** |
| A | Neusilin® UFL2 | ≥ 0 | amorphous | amorphous | amorphous | amorphous |
| B | Neusilin® US2 | ≥ 0 | amorphous | amorphous | amorphous | amorphous |

ΔΔm is the difference between the mass difference Δm(desorption) at 75 % relative humidity and 25°C and the mass difference Δm(adsorption) at 75 % relative humidity and 25°C. Neusilin UFL2^{®}: neutral magnesium aluminometasilicate commercialized by Fuji Chemical Industry Col., Ltd. (powder with a trapped bulk density of 0.10-0.17 g/mL); Neusilin US2^{®}: neutral magnesium aluminometasilicate commercialized by Fuji Chemical Industry Col., Ltd (granule with a trapped bulk density of 0.16-0.22 g/mL).

### Example 4-2: weight ratio betrixaban maleate / silicon-based compound 1:2

To betrixaban maleate (about 35 mg, prepared according to the procedure disclosed in WO2011/084519, Example 2) and silicon-based compound (about 70 mg) according to Table 6 were added 8 mL of acetonitrile/water solution (volume ratio 50:50), leading to a solution of betrixaban maleate with suspended silicon-based compound. The suspension was homogeneously frozen in a bath of liquid nitrogen and lyophilized at a pressure of from 0 to 2 mbar, yielding amorphous solid dispersions of betrixaban maleate. The so obtained solid dispersions were open stored under accelerated stability conditions (temperature: 40°C, relative humidity: 75%) for six weeks. The samples were periodically analysed by powder X-ray diffraction and the results are summarized in Table 6.

**Table 6: Accelerated stability test of solid dispersions of amorphous betrixaban maleate and silicon-based compound (weight ratio API / silicon-based compound 1:2)**

| **Example** | **Carrier** | | **Physical form** | **PXRD analysis after storage at 40°C and 75% RH for** | | |
|---|---|---|---|---|---|---|
| | **Compound** | ΔΔm (%) | | **1 week** | **3 weeks** | **6 weeks** |
| A | Neusilin® UFL2 | ≥ 0 | amorphous | amorphous | amorphous | amorphous |
| B | Neusilin® US2 | ≥ 0 | amorphous | amorphous | amorphous | amorphous |
| C | Syloid^{®} 72FP | ≥ 0 | amorphous | amorphous | amorphous | amorphous |

ΔΔm is the difference between the mass difference Δm(desorption) at 75 % relative humidity and 25°C and the mass difference Δm(adsorption) at 75 % relative humidity and 25°C. Neusilin UFL2^{®}: neutral magnesium aluminometasilicate commercialized by Fuji Chemical Industry Col., Ltd. (powder with a trapped bulk density of 0.10-0.17 g/mL); Neusilin US2^{®}: neutral magnesium aluminometasilicate commercialized by Fuji Chemical Industry Col., Ltd (granule with a trapped bulk density of 0.16-0.22 g/mL); Syloid^{®} 72FP: neutral micronized synthetic amorphous silica gel commercialized by Grace.

### Example 4-3: weight ratio betrixaban maleate/ silicon-based compound 1:3

To betrixaban maleate (about 30 mg, prepared according to the procedure disclosed in WO2011/084519, Example 2) and silicon-based compound (about 90 mg) according to Table 7 were added 8 mL of acetonitrile/water solution (volume ratio 50:50), leading to a solution of betrixaban maleate with suspended silicon-based compound. The suspension was homogeneously frozen in a bath of liquid nitrogen and lyophilized at a pressure of from 0 to 2 mbar, yielding amorphous solid dispersions of betrixaban maleate. The so obtained solid dispersions were open stored under accelerated stability conditions (temperature: 40°C, relative humidity: 75%) for six weeks. The samples were periodically analysed by powder X-ray diffraction and the results are summarized in Table 7.

**Table 7: Accelerated stability test of solid dispersions of amorphous betrixaban maleate and silicon-based compound (weight ratio API / silicon-based compound 1:3)**

| **Example** | **Carrier** | **Physical form** | **PXRD analysis after storage at 40°C and 75% RH for** | | |
|---|---|---|---|---|---|
| | | | **1 week** | **3 weeks** | **6 weeks** |
| A | Neusilin® UFL2 | amorphous | amorphous | amorphous | amorphous |
| B | Neusilin® US2 | amorphous | amorphous | amorphous | amorphous |
| C | Syloid® 72FP | amorphous | amorphous | amorphous | amorphous |

Neusilin UFL2^{®}: neutral magnesium aluminometasilicate commercialized by Fuji Chemical Industry Col., Ltd. (powder with a trapped bulk density of 0.10-0.17 g/mL); Neusilin US2^{®}: neutral magnesium aluminometasilicate commercialized by Fuji Chemical Industry Col., Ltd (granule with a trapped bulk density of 0.16-0.22 g/mL); Syloid^{®} 72FP: neutral micronized synthetic amorphous silica gel commercialized by Grace.

According to the results presented in Table 7, solid dispersions of amorphous betrixaban maleate prepared with Neusilin^{®} UFL2, Neusilin^{®} US2 or Syloid^{®} 72FP (75 weight-% silicon-based carrier loading) are stable upon storage at 40°C and 75% RH.

## Claims

1. A solid dispersion comprising amorphous betrixaban maleate and a pharmaceutically acceptable carrier selected from the group consisting of organic polymers, silicon-based inorganic compounds and mixtures thereof.

2. The solid dispersion according to claim 1, wherein in the adsorption-desorption isotherm of the pharmaceutically acceptable carrier, the mass difference Δm(desorption) at 75 % relative humidity and 25°C is greater than or equal to the mass difference Δm(adsorption) at 75 % relative humidity and 25°C, determined according to dynamic vapour sorption measurement.

3. The solid dispersion according to claim 1 or 2, wherein the pharmaceutically acceptable carrier is an organic polymer.

4. The solid dispersion according to claim 1 to 3, wherein the organic polymer is a hydrophilic organic polymer.

5. The solid dispersion according to claim 1 or 4, wherein the organic polymer is a cellulose derivative selected from the group consisting of alkylcellulose, preferably methylcellulose, ethylcellulose, or propylcellulose; hydroxyalkylcellulose, preferably hydroxymethylcellulose, hydroxyethylcellulose, or hydroxypropyl cellulose; hydroxyalkyl alkylcellulose, preferably hydroxyethyl methylcellulose (HEMC), or hydroxypropyl methylcellulose (HPMC); carboxyalkyl cellulose, preferably carboxymethylcellulose (CMC), carboxymethyl hydroxyethylcellulose (CMHEC), hydroxyethyl carboxymethylcellulose (HECMC); sodium carboxymethylcellulose, cellulose acetate phthalate (CAP), hydroxypropyl methylcellulose acetate (HPMCA), hydroxypropyl methylcellulose phthalate (HPMCP), hydroxypropyl methylcellulose acetate succinate (HPMCAS), and a mixture of two or more thereof.

6. The solid dispersion according to claims 1 to 5, wherein the organic polymer is hydroxypropyl methylcellulose.

7. The solid dispersion according to claim 1 or 2, wherein the pharmaceutically acceptable carrier is a silicon-based inorganic compound.

8. The solid dispersion according to claim 1, 2 or 7, wherein the silicon-based inorganic compound is selected from the group consisting of silica, silicates, and a combination of two or more thereof.

9. The solid dispersion according to claim 1, 2, 7 or 8, wherein the silicon-based inorganic compound has a pH in the range of from 6.0 to 9.0, preferably in the range of from 6.3 to 8.5, more preferably in the range of from 6.6 to 8.0.

10. The solid dispersion according to any of the preceding claims, wherein the weight ratio of betrixaban maleate and the pharmaceutically acceptable carrier is from 1:1 to 1:10.

11. The solid dispersion according to any one of the preceding claims comprising at most 20%, preferably at most 10%, more preferably at most 5%, even more preferably at most 2% and most preferably at most 1% by weight of any crystalline form of betrixaban maleate.

12. A pharmaceutical composition comprising a solid dispersion according to any one of the preceding claims and one or more pharmaceutically acceptable excipients.

13. A process for the preparation of the solid dispersion of claims 1 to 11 comprising the steps of
a) providing betrixaban maleate; and
b) dissolving or dispersing betrixaban maleate and the pharmaceutically acceptable carrier in a solvent to form a mixture; and
c) removing the solvent to provide the solid dispersion.

14. A solid dispersion according to claims 1 to 11 or a pharmaceutical composition according to claim 12 for use as a medicament.

15. A solid dispersion according to claims 1 to 11 or a pharmaceutical composition according to claim 12 for use in the treatment of disease conditions **characterized by** undesired thrombosis.
